# EUROPEAN PATENT SPECIFICATION

(11) **EP 2 497 487 B1**
(45) Date of publication and mention of the grant of the patent: **07.08.2013**
(21) Application number: 12170754.1
(22) Date of filing: 01.07.2004
(51) Int. Cl.: A61K 38/46

(54) **Method for treating diabetes and atherosclerosis associated with changes of qualitative and/quantitative composition of blood extracellular DNA**
VERFAHREN ZUR BEHANDLUNG VON DIABETES UND ATHEROSKLEROSE IM ZUSAMMENHANG MIT VERÄNDERUNGEN DER QUALITATIVEN UND/ODER QUANTITATIVEN ZUSAMMENSETZUNG VON EXTRAZELLULÄRER DNA IM BLUT
METHODE DE TRAITEMENT DU DIABÈTE ET DE L'ATHÉROSCLÉROSE S'ACCOMPAGNANT D'ALTERATIONS DE LA COMPOSITION QUALITATIVE ET/OU& xA;QUANTITATIVE DE L'ADN EXTRACELLULAIRE DU SANG

(30) Priority: 14.07.2003 WO PCT/RU03/00304; 12.03.2004 RU 2004108057
(43) Date of publication of application: 12.09.2012
(62) Divisional of application: 04775224.1
(73) Proprietor: CLS Therapeutics Limited, Guernsey GY1 3DR (GB)
(72) Inventor: Genkin, Dmitry Dmitrievich, 197110 St. Petersburg (RU); Tets, Victor Veniaminovich, 196066 St. Petersburg (RU); Tets, Georgy Victorovich, 191040 St. Petersburg (RU)
(74) Representative: Gulde Hengelhaupt Ziebig & Schneider

(56) References cited:
- DE-A1- 4 024 530
- SUGIHARA S ET AL: "Deoxyribonuclease treatment prevents blood-borne liver metastasis of cutaneously transplanted tumour cells in mice", BRITISH JOURNAL OF CANCER, NATURE PUBLISHING GROUP, LONDON, GB, vol. 67, no. 1, 1 January 1993 (1993-01-01), pages 66-70, XP008086562, ISSN: 0007-0920
- DAVIS J C JR ET AL: "Recombinant human Dnase I (rhDNase) in patients with lupus nephritis", LUPUS, BASINGSTOKE, GB, vol. 8, no. 1, 1 January 1999 (1999-01-01) , pages 68-76, XP009124143, ISSN: 0961-2033, DOI: 10.1191/096120399678847380
- FUNAKOSHI A ET AL: "CLINICAL INVESTIGATION OF SERUM DNASE 2. CLINICAL STUDIES OF SERUM DNASE ACTIVITY IN PANCREATIC DISEASE", GASTROENTEROLOGIA JAPONICA, JAPANESE SOCIETY OF GASTROENTEROLOGY, TOKYO, vol. 14, no. 5, 1 January 1979 (1979-01-01), pages 436-440, XP009161108, ISSN: 0435-1339
- BOTTO NICOLETTA ET AL: "Elevated levels of oxidative DNA damage in patients with coronary artery disease", CORONARY ARTERY DISEASE, CURRENT SCIENCE LTD, vol. 13, no. 5, 1 August 2002 (2002-08-01) , pages 269-274, XP009161106, ISSN: 0954-6928

## Description

### Technical field

The invention reveals to medicine and veterinary and refers to DNAse enzyme which is used for treatment of atherosclerosis and diabetes.

### Background art

The main method of therapy of diseases caused by bacteria, fungi and protozoa are antibiotics and chemotherapy (see Merck Manual of Diagnosis and Therapy; 16the Edition). The main way of atherosclerosis' drug therapy is therapy with statins' group's compounds inhibiting the cholesterol synthesis (see New Concepts and Paradigms in Cardiovascular Medicine: The Noninvasive Management of Coronary Artery Disease, K. Lance Gould, THE AMERICAN JOURNAL OF MEDICINE, Volume 104, June 22, 1998, 2s-17s.)

Therapy of diabetes mellitus consists from three main approaches - insulin therapy, drugs increasing insulin' secretion by pancreas, drugs increasing sensitivity of tissues to the insulin or the same increasing glucose' utilization by tissues (Pharmacological Management of Diabetes: Recent Progress and Future Perspective in Daily Drug Treatment, Gérard Emilien et.al., Pharmacol. Ther. Vol. 81, No. 1, pp. 37-51, 1999).Therapy of type IV hypersensitivity is based on immunosuppressive and immunomodulating therapy (see Therapeutic Immunosupression, ed.A.W.Thomson, Ser.Immunology and Medicine vol.29, Kluwer Acad.Publishers, Dordrecht, 2001).

The diseases caused by mutations in somatic genes and accompanied by somatic mosaicism development have got no etiological therapy, see Youssoufian H, Pyeritz RE. Mechanisms and Consequences of Somatic Mosaicism in Humans,. Nature Reviews Genetics, 2002;3:748-758.

The drug resistance is considered as the main problems of the antibiotic therapy of bacterial infection. The circulation of antibioticresistant strains and appearance of new ones in the process of the treatment (for example as a result of biofilms' formation in the patient's organism) are the main cause of therapy's inefficiency (The use and resistance to antibiotics in the community. Cizman M, Int J Antimicrob Agents, 2003, Apr 21: pp.297-307).

At the present time it is universally recognized that problem of antibiotic resistance has a character of global threat (Mechanisms of antimicrobial resistance: their clinical relevance in the new millennium. Sefton A.M., Drugs, 2002, vol. 62: 557-66) and it asks for development of new original antibiotics and new method with non-antibiotic mechanism of effect on the infectious process. For example vancomycin is used for infectious' treatment caused be Gram positive cocci resistant to penicillin and cephalosporin. Main disadvantages of Vancomycin are increasing of circulating number of Vancomycin-resistant strains; high toxicity; relatively narrow spectrum of activity (The threat of vancomycin resistance. PerlTM, Am J Med 1999 May 106:26S-37S).

The aforesaid data indicate that development new effective, low toxic, method demonstrated broad-spectrum activity against all species bacteria including antibiotic -resistant strains is still an extremely important task. Problems of antibiotic therapy and chemotherapy of infectious caused by fungi and protozoa are close to those of bacterial infection treatment; for example, when an established drug - amphotericin is used (Antifungal drug resistance to azoles and polyenes, Mar Masiá Canuto et.al., The Lancet Infectious Diseases, Volume 2, Issue 9, 1 September 2002, Pages 550-563; A systematic review of the antifungal effectiveness and tolerability of amphotericin B formulations, Jane P. Barrett et.al., Clinical Therapeutics,Volume 25, Issue 5, May 2003, Pages 1295-1320).

Atherosclerosis is a systemic disease that is accompanied by formation of specific atherosclerotic plaques in large and middle sized artery walls. Depends on the localization, stage and size of atherosclerotic plaques the disease has different clinical signs (angina, stroke and so on). The signs especially associated with organ dysfunction caused by systemic atherosclerosis are cured by drug therapy or by surgical operation. There is no cure for Aterosclerosis by drug therapy methods as for any systemic disease. An established method of prevention that delays the disease progression is therapy with inhibitors of 3 3-hydroxy-3-methylglutaryl-CoA (HMG CoA) reductase (Lovastatin, Parvastatine e.t.c.) leading to the inhibition of endogenous cholesterol synthesis and increasing of clearance of low density lipoproteins of blood plasma and it attenuates atherosclerosis development (New Concepts and Paradigms in Cardiovascular Medicine: The Noninvasive Management of Coronary Artery Disease, K. Lance Gould, THE AMERICAN JOURNAL OF MEDICINE, Volume 104, June 22, 1998, 2s-17s). Disadvantages of such treatment are adverse effects (A safety look at currently available statins., Moghadasian MH, Expert Opin Drug Saf 2002 Sep 1:pp.269-74) and limited efficacy (Statins: balancing benefits, efficacy and safety., Clearfield MB, Expert Opin Pharmacother, 2002, May 3:pp.469-77).

The main cause of disablement and death of patients with diabetes mellitus type 1 and 2 are complications associated with microangiopathy and macroangiopathy development. It is considered that effective metabolic control of glucose level (maintenance of glucose level and glycated hemoglobin level within the normal limits) prevents development of complications. The insulin therapy including intensive insulin therapy is the method of choice when it is impossible to reach metabolic control with other drugs (Outpatient insulin therapy in type 1 and type 2 diabetes mellitus: scientific review, DeWitt DE, Hirsch IB,JAMA, 2003, May 289:pp.2254-64).

But even if high-dose insulin therapy are used the risk of developing the complications including fatal ones is still sufficiently high (Cause-specific mortality in a population with diabetes: South Tees Diabetes Mortality Study, Roper NA, et al, Diabetes Care, 2002, Jan 25:pp.43-8). In accordance with above-mentioned the task of development of new methods of type I and type II diabetes mellitus therapy including the complication prevention methods is still topical and generally recognized.

One of the established clinical methods of treatment of r delayed-type hypersensitivity reactionsis administration of Cyclosporine A peptide (Therapeutic Immunosupression, ed.A.W.Thomson, Ser. Immunology and Medicine vol.29, Kluwer Acad. Publishers, Dordrecht, 2001). The well - known drawbacks of this method are severe adverse effects namely nephrotoxicity, hypertension and high risk of infections' development (Cyclosporine: mechanisms of action and toxicity., Graham RM, Cleve Clin J Med, 1994, Jul-Aug 61:pp.308-13). Another problem is loss the efficacy during long-term treatment, that reveals itself in the increasing risk for transplant rejection (Renal transplantation, past, present and future, Ponticelli C, et al, J Nephrol, 1999, Jul-Aug 12 Suppl 2:S105-10).Thus for treatment accompanied by qualitative and/or quantitative changes of blood extracellular DNA the wide spectra of different method are used that have got similar disadvantages: toxicity, adverse effects; low efficacy of therapy. At the same time in the real clinical practice these diseases often accompany each other. For example the therapy delayed-type hypersensitivity reactions with immunosuppressive drugs multiplies the risk of infectious diseases (Recent advances in the diagnosis and management of infection in the organ transplant recipient. Tolkoff-Rubin NE, Rubin RH; Semin Nephrol 2000 Mar 20:148-63.); atherosclerosis is a very common complication of diabetes mellitus (Diabetes and atherosclerosis: epidemiology, pathophysiology, and management, Beckman JA, Creager MA, Libby P;JAMA 2002 May 287:2570-81) and is often accompanied by systemic infectious process (Infection and atherosclerosis: potential roles of pathogen burden and molecular mimicry., Epstein SE, Zhu J, Burnett MS, Zhou YF, Vercellotti G, Hajjar D, Arterioscler Thromb Vasc Biol 2000 Jun 20:1417-20.); a number of diabetes types develops as a result of delayed-type hypersensitivity reaction (Evidence of islet cell autoimmunity in elderly patients with type 2 diabetes., Pietropaolo M, Barinas-Mitchell E, Pietropaolo SL, Kuller LH, Trucco M, Diabetes 2000 Jan 49:32-8), or during the infectious process (Systemic diseases caused by oral infection. Li X, Kolltveit KM, Tronstad L, Olsen I, Clin Microbiol Rev 2000 Oct 13:547-58), and leads to the high risk of infections' development (Diabetes and the risk of infection-related mortality in the U.S.,Bertoni AG, Saydah S, Brancati FL, Diabetes Care 2001 Jun 24:6 1044-9).

US 6,391,607 B1 relates to amino acid sequence variants of human DNase I that have increased DNA-hydrolytic activity. It provides nucleic acid sequences encoding such hyperactive variants, thereby enabling the production of these variants in quantities sufficient for clinical use. Further it relates to pharmaceutical compositions and therapeutic uses of hyperactive variants of human DNase I.

According to DE 40 24 530 A1 viruses and viral diseases are controlled in humans and animals by treatment with nucleases (I) isolated from bovine pancreas. (I) also include ribonucleases and deoxyribonucleases, specifically DNase I.

SUGIHARA S ET AL: describe in BRITISH JOURNAL OF CANCER, NATURE PUBLISHING GROUP, LONDON, GB, vol. 67, no. 1, 1 January 1993 (1993-01-01), pages 66-70, XP008086562 ISSN: 0007-0920 that a deoxyribonuclease treatment prevents blood-borne liver metastasis of cutaneously transplanted tumour cells in mice.

DAVIS J C JR ET AL: describe in LUPUS, BASINGSTOKE, GB, vol. 8, no. 1, 1 January 1999 (1999-01-01), pages 68-76, XP009124143 ISSN: 0961-2033 the use of recombinant human Dnase I (rhDNase) in patients with lupus nephritis.

### Disclosure of the invention

The solving of the aim of development of high-performance and low-toxic method of treatment of the diseases diabetes and atherosklerosis that are accompanied by quantitative and/or qualitative change of composition of blood plasma extracellular DNA is the basis of this invention.

According to the invention this task is resolved by introducing of blood extracellular DNA destroying agent into a systemic blood circulation for treating atherosclerosis and diabetes. As the agent destroying blood extracellular DNA DNase enzyme can be introduced in systemic circulation: enzyme DNAase can be introduced in systemic circulation in doses that provide change of electroforetic profile of blood extracellular DNA that can be detected by puls-gel-electroforesis, whereby the DNase enzyme can be administrated at doses and regimens that can provide blood DNA-hydrolytic level measured in blood plasma and exceeded 150 Kuntz units per liter of plasma, and this level can be supported for more than 12 hours during 24 hours in total.

Development of diabetes and atherosclerosis is accompanied by quantitative and/or qualitative change of blood extracellular DNA, but in source of available data, there are no knowledge about genetic repertoire of extracellular blood DNA of patients with above-mentioned diseases, about biological role of extracellular blood DNA in these diseases and about potential therapeutic effect of blood extracellular DNA destroying with the purpose of treatment of these diseases; so, taking into account all aforesaid, the invention conformances to requirements of "novelty" criteria (N).

As the applicant established, the extracellular blood DNA of patients with foregoing diseases contains the unique quantitative and qualitative repertoire of genes and regulating genetic elements which great differ from the repertoire of DNA which is described in human genome. In contrast to inracellular DNA the extracellular DNA of these patients contains mainly unique human genes. Extracellular bacterial and fungal DNA was found out in biofilms' matrix and blood plasma of the infected human.

It was established that blood extracellular DNA including extracellular DNA of bacteria, fungi and protozoa promotes the development of diseases listed above.

It was established that destruction of blood extracellular plasma DNA leads to therapeutic effect on the diseases listed above.

Aforesaid new characteristics of the claimed invention are based on new ideas about mechanisms of the described diseases. In this way the claimed method conformances to requirements of "invention step" criteria (IS).

### Brief description of the drawings

As set for below the invention has been explained by detailed description of embodiments without references to drawings.

### Preferred embodiment

The claimed inventive method is realized by following:

### Materials and methods.

The following agents that destroy extracellular blood DNA were used: bovine pancreatic DNase (Sigma and Samson-Med), recombinant human DNase I (Gentech), DNA-hydrolyzing anti-DNA antibodies isolated from the blood of patients with lupus erythematosus according to Shuster A.M. (Shuster A.M. et.al., Science,v.256,1992, pp.665-667).

Extracellular DNA from blood plasma was isolated as follows: fresh plasma with (no more than 3-4 hours after sampling) with an added anticoagulant (sodium citrate) was centrifuged on Ficoll-PlaquePlus (Amersham-Pharmacia) during 20 minutes at 1500 g at room temperature. ½ of plasma was detached, not affecting the rest of cells on the Ficoll pillow, and further centrifuged at 10000 g during 30 min for separation from cell fragments and debris. Supernatant was detached, without affecting of the sediment, and was toped up to 1% of sarkosil, 50MM tris-HCl, pH 7,6, 20 MM EDTA, 400 MM NaCl, and than mixed with equal volume of phenol-chloroform(1:1) mixture. The prepared emulsion was incubated during 2 hours at t=65°C, then phenol-chloroform mixture was separated by centrifuging (500g during 20 minutes, room temperature).

The procedure of deproteinization with phenol - chloroform mixture was repeated 3 times, and then the water phase was processed with chloroform and diethyl ether. Separation from organic solvents was made by centrifugation at 5000g during 15 minutes). Then equal volume of isopropanol was added to resulting aqueous phase and the mixture was incubated overnight at 0°C. After sedimentation the nucleic acids were separated by centrifugation at 10000g during 30 minutes. The sediment of nucleic acids was dissolved in of 10MM tris-HCl buffer, pH 7, 6 with 5 MM EDTA, and inflicted to the CsCl gradient (1M, 2.5M, 5.7M) in test-tube for rotor SW60Ti. The volume of DNA solution was 2 ml, volume of each step of CsCl was 1 ml. Ultracentrifugation was conducted in L80-80 (Beckman) centrifuge during 3 hours at 250000g. DNA was collected from the surface of each gradient step into fractions. These fractions were dialyzed during 12 hours (t=4°C). Presence of DNA in fractions was determined by agar electrophoresis and DNA was visualized by ethidium bromide staining. The amount of DNA was determined with spectrophotometer (Beckman DU70) in cuvet (100 mcl) at wavelength of 220-230 nm.

### Example 1. Influence of DNase therapy on the viability of pancreatic beta-cells and endothelium of aorta.

Human recombinant DNase I (Gentech) was used. ß□□ cells of human embryonic pancreas and endothelial cells of human aorta were used for primary cell culture formation. DNA isolated from plasma of patient with severe form of diabetes mellitus type 2 that was complicated by atherosclerosis (0.0025 mkg on 1 ml of culture media) was added to one of the experimental series in cell culture 24 hours after passage and DNA extracted from the blood of the same patient but treated by DNase (1 mkg/ml; 37C; 30 minutes) was added to the second series of cell culture. The number of viable cells was counted using the trypan blue uptake technique in 24 hours.

Results of the experiment are presented in table 4:

**Table 4**

| Percentage of viable cells 48 hours after their cultivation (in percents). | | | |
|---|---|---|---|
| Cells | Control | DNA of patient | DNA of patent treated by DNse |
| ß□□ cells | 73% | 43% | 61% |
| Endotelium | 62% | 35% | 55% |

Thus extracellular blood plasma DNA of patient with severe form of diabetes mellitus type 2 and atherosclerosis negatively influence both on the normal pancreatic β □-cells and on the normal endothelial cells. Destruction of the patient' blood extracellular DNA prevents development of negative influence according to the claimed method.

### Example 2. Atherosclerosis' treatment.

54-years-old man has been admitted to the hospital in grave condition complaining on intensive pain in abdomen, diarrhea, intensive pain in legs that appear during walking, loss of weight. Diabetes mellitus type 2 was diagnosed 12 years ago and glybencamyde was prescribed. Pain in epigastrium after food intake appeared 15 months ago. Antacids were prescribed but pain continued to increase and steatorrhea appeared in the last 3 months. Because of intensive pain syndrome anorexia has developed in a couple of days before hospitalization. Considerable exhaustion (body weight was 44 kg; body weight loss was 28 kg for the last 5 months) and absence of aorta' pulsation on legs were found out during examination.

No organic changes were observed during gastroduodenoscopy and colonoscopy. Data of ECG was not changed pathologically. Moderate increase of cholesterol level and low-density lipoprotein fraction was observed in blood analysis. Glycated hemoglobin' level was 11%. Partial occlusion of aorta below renal artery (70%), partial occlusion of iliac arteries (90%), total occlusion of upper and lower mesenteric artery were observed on aortography. Conservative therapy was chosen due to absence of possibility to use surgical methods of treatment. Intensive parenteral nutrition was started. Insulin therapy was prescribed. The antiaggregants' therapy has being carried out. By patient's consent daily intravenous infusions of bovine pancreatic DNase at dose 800 mg/day (1 600 000 Kunitz units) divided to 4 two-hour administration were started on the 7^{th} day after the beginning of the therapy patient was allowed to take dietetic food. Pain syndrome disappeared. Patient has received full value enteral nutrition to the 20th day of therapy. General state has improved, body weight has increased. On the 45th day of the therapy angiography as a part preoperative study was done. Decrease of occlusion level of aorta and iliac arteries on 20% and 30% correspondently, and appearance of blood circulation in upper and lower mesenteric artery (occlusion 80%) was observed.

Samples of extracellular DNA of this patient were cloned by the method that allows to construct not amplified plasma library of such DNA with representativeness up to million clones with average size 300-500 base.

Isolated according to above-mentioned method, DNA was deproteinized with the use of proteinase K (Sigma) at 65°C for tightly-bound proteins elimination. After deproteinization DNA was treated by phenol-chloroform at 65°C and sedimented by 2.5 volumes of ethanol during night. After it DNA was processed by EcoRI restrictase during 3 hours or by Pfu polymerase (Stratagene) at the presence of 300 mkM of all desoxynucleothydethreephosphates for "sticky" edges elimination. Completed DNA was phosphorylated by polynucleotide kinase T4 (30U, 2 hours). Received samples/preparations were ligated in Bluescript (Stratagene), plasmid digested by EcoR1 or PvuII accordingly and dephosphorylated by alkaline phosphatase CIP (Fermentas) during 1 hour. 1 mkg of vector and 0.1-0.5 mkg of serum DNA were usually used for ligation. The ligation was done by Rapid Ligation Kit (Roche) use for 10 hours at 16°C. Volume of ligase mixture was 50 mkl. Ligated library was transformed into DH12S (Life Technologies) cells with electroporator (BioRad) use. For transformation of one library 12-20 electroporation cuvettes were used. Dilutions of the library at concentrations 10⁻⁴, 10⁻⁵ and 10⁻⁶ were plated for control on dishes with 1,5% agar and LB media. In both cases library's representativeness was approximately 2-3x10⁶ clones.

Analysis of randomly chosen clones with length from 300 up to 1000 base pair units from the library that was obtained from the blood extracellular plasma DNA of patient before treatment has indicated that 56 from 75 clones are unique fragments of human DNA. The function or the product of correspondent gene was identified for 11 genes by HumanGeneBank.

| | |
|---|---|
| Gene or corresponding protein product | Reported role in Atherosclerosis and Diabetes |
| Neutral endopeptidase | At atherosclerosis its activity is increased in endothelial cells, nonstriated muscle cells, stromal cells of artery' intima. Decreasing of its activity can decrease lipids accumulation in vessels wall. |
| Muskelin 1 | Is mediator of cell response on thrombospondin 1. thrombospondin 1 - mediated processes are pathophysiological components of atherosclerotic affection of artery wall. |
| Nf-kappaB | At hyperglycemia and atherosclerosis its activity is increased in cells of artery wall. |
| Transient receptor potential cation channel | |
| Phospholipase C, epsilon | Induces expression of receptors of low density lipoproteins. |
| CRTL1: cartilage linking protein 1 | |
| 17kD fetal brain protein | |
| Nicotinamide nucleotide transhydrogenase | |
| BAI3: brain-specific angiogenesis inhibitor | |
| GAD2: glutamate decarboxylase 2 | One of the main autoantigens at diabetes type 1. |
| E-selectin | High level of expression is a risk factor of angiopathy' development at diabetes type 2. |

Analysis of 50 clones randomly chosen from the library obtained from the extracellular blood plasma DNA of patient on the 21^{st} day after the beginning of treatment has shown that more than 90% of revealed clone consequences are short fragments of repeating human DNA, in the main alpha-satellite DNA. Changes in electroforetic profile of blood extracellular DNA that is evaluated by pulse gel electrophoresis were registered at the same time.

Thus DNase use according to the claimed method possess therapeutic effect at atherosclerosis.

### Example 3. Diabetes mellitus treatment.

Poor metabolic control of diabetes mellitus, that is revealed by high level of glycated hemoglobin in blood and low sensitivity to insulin makes it necessary to use high doses of insulin and are main predisposing factors to the complications' development.

46-years-old patient suffers from diabetes mellitus type 2 for 3 years. Because of the unsuccessfully results of oral antidiabetic compounds that were not able to decrease blood glucose level, the human recombinant short-acting insulin was prescribed at 0.3 U/kg (21U/day). The level of glycated hemoglobin in blood was still to be high (more than 10%), symptoms of diabetic angiopathy and polyneuropathy and decrease in sharpness of vision have appeared. Daily insulin demand has increased up to 1.2 U/kg (84 U/day). Intramuscular injections of bovine pancreatic DNase at 200 mg/day (2 times a day) for 4 months were prescribed to the patient. To the end of treatment course the patient was better, level of glycated hemoglobin in blood was diminished and daily dose of insulin was also diminished in two times. The results are presented in table 5.

**Table 5**

| The effect of DNase treatment on patient' metabolic indexes. | | | |
|---|---|---|---|
| | Before treatment | Three months after the beginning of treatment | Four months after the beginning of treatment |
| Glycated hemoglobin (%) | 13,2 | 10,1 | 7,2 |
| Necessity for insulin U/kg body weight | 1,2 | 0,9 | 0,6 |
| Amount of extracellular blood plasma DNA (%). | 100 | 85 | 70 |

Amount of blood extracellular DNA was estimated by densitometry of electrophoretic band. DNA amount before treatment was taken as 100%.

Thus DNase use at diabetes possesses therapeutic effect according to the claimed method.

### Industrial applicability

For the realization the methods there were used well-known materials and equipment manufactured in plant conditions and according to aforesaid the invention conformances to requirements of "industrial applicability" criteria (IA).

## Claims

1. DNAse enzyme for use in treating diabetes and atherosclerosis associated with changes of qualitative and/or quantitative composition of blood extracellular DNA, wherein the DNAse is introduced into the systemic blood circulation.

2. DNAse enzyme according to claim 1 wherein the DNAase enzyme is introduced in doses sufficient to provide blood extracellular DNA electrophoretic profile change, wherein the DNAase enzyme is introduced in doses and regimens which provide blood plasma DNA-hydrolytic activity measured in blood plasma, to exceed 150 Kunitz units per litre of plasma during more than 12 hours in total within 24 hours.

## Patentansprüche

1. DNase-Enzym zur Verwendung bei der Behandlung von Diabetes und Atherosklerose, die mit Änderungen der qualitativen und/oder quantitativen Zusammensetzung von extrazellulärer DNA im Blut verbunden sind, wobei die DNase in den systemischen Blutkreislauf eingebracht wird.

2. DNase-Enzym nach Anspruch 1, wobei das DNase-Enzym in hinreichenden Dosen eingebracht wird, um eine Änderung des elektrophoretischen Profils extrazellulärer DNA im Blut zu ergeben, wobei das DNase-Enzym in Dosen und Regimes eingebracht wird, die eine im Blutplasma gemessene Blutplasma-DNA-Hydrolyseaktivität von mehr als 150 Kunitz-Einheiten pro Liter Plasma im Laufe von insgesamt mehr als 12 Stunden innerhalb 24 Stunden ergeben.

## Revendications

1. Enzyme DNase destinée à une utilisation dans le traitement du diabète et de l'athérosclérose associée à des changements de composition qualitative et/ou quantitative d'ADN extracellulaire dans le sang, dans laquelle la DNase est introduite dans la circulation sanguine systémique.

2. Enzyme DNase selon la revendication 1, moyennant quoi l'enzyme DNase est introduite dans des doses suffïsantes pour modifier le profil électrophorétique d'ADN extracellulaire dans le sang et l'enzyme DNase est introduite dans des doses et des schémas posologiques qui procurent une activité hydrolyticlue d'ADN de plasma sanguin, mesurée dans le plasma sanguin, pour dépasser 150 unités Kunitz par litre de sang pendant plus de °i2 heures au total dans un délai de 24 heures.
